# EUROPEAN PATENT SPECIFICATION

(11) **EP 2 668 904 B1**
(45) Date of publication and mention of the grant of the patent: **28.11.2018**
(21) Application number: 13169908.4
(22) Date of filing: 30.05.2013
(51) Int. Cl.: A61B 6/14, A61B 1/24, A61B 5/00, A61B 1/04

(54) **SPECTRAL FILTER FOR AN INTRA-ORAL IMAGING SYSTEM**
SPEKTRALFILTER FÜR EIN INTRAORALES BILDGEBUNGSSYSTEM
FILTRE SPECTRAL POUR SYSTÈME D'IMAGERIE INTRAORALE

(30) Priority: 30.05.2012 US 201213484156
(43) Date of publication of application: 04.12.2013
(73) Proprietor: Ormco Corporation, Orange, CA 92867 (US)
(72) Inventor: DILLON, Robert F, Bedford, NH 03110 (US); ANDREIKO, Craig A., Alta Loma, CA 91737 (US)
(74) Representative: Findlay, Alice Rosemary

(56) References cited:
- EP-A1- 1 745 736
- WO-A1-2011/011193
- US-A1- 2008 090 198
- US-B1- 6 373 573

## Description

The disclosure relates to a system, method, and a computer readable storage medium for a spectral filter for an intra-oral imaging system.

Spectral filtering may be used to select or eliminate information from an image based on the wavelength of the information. Spectral filtering is usually performed by passing light through a glass or plastic window that has been specially treated to 'transmit or absorb or reflect some wavelengths.

An intra-oral imaging system is a diagnostic equipment that allows a dental practitioner to see the inside of patient's mouth and display the topographical characteristics of teeth on a display monitor. Certain three-dimensional (3D) intra-oral imagers may be comprised of an intra-oral camera with a light source. The 3D intra-oral imager may be inserted into the oral cavity of a patient by a dental practitioner. After insertion of the intra-oral imager into the oral cavity, the dental practitioner may capture images of visible parts of the teeth and the gingivae.

The 3D intra-oral imager may be fabricated in the form of a slender rod that is referred to as a wand or a handpiece. The wand may be approximately the size of a dental mirror with a handle that is used in dentistry. The wand may have a built-in light source and a video camera that may achieve an imaging magnification, ranging in scale from 1 to 40 times or more. This allows the dental practitioner to discover certain types of details and defects of the teeth and gums. The images captured by the intra-oral camera may be displayed on a television or a computer monitor.

The wand may be attached or linked to a computer and a display monitor. The wand, the computer, and the display monitor may all be placed in the proximity of the patient before the dental practitioner places the tip of the wand inside the oral cavity of the patient and starts acquiring images. The acquired images may be displayed on the display monitor and may also be saved on a storage device. Furthermore, the acquired images may be transmitted to a remote computational device for additional processing.

EP 1 745 736 discloses having an irradiation means comprising an exciting light emission section emitting an exciting light for causing a lesion to radiate fluorescence, and an illumination light emission section emitting a light for illuminating the periphery of the lesion. The irradiating means can simultaneously irradiate the illumination light and the exciting light.

The invention is defined by the claims.

In certain embodiments, the light of other wavelengths are caused via fluorescence of the structures that exist in the oral cavity of patient, wherein the fluorescence includes auto-fluorescence of teeth. Furthermore, removal of the spectral filter from the imaging device causes errors in generation of the three-dimensional structures because the other wavelengths are spurious wavelengths that are unsuitable for performing the metrology.

In additional embodiments, the wand has a proximal end and a distal end, wherein the imaging device is coupled to the distal end of the wand. The electrical signals are digitized and transmitted wirelessly for processing via a processor.

In yet further embodiments, the imaging device is a digital camera, wherein the spectral filter is removable from the imaging device.

In certain additional embodiments, the metrology wavelength is less than 405 nanometers.

In certain embodiments, the light of the metrology wavelength is transmitted through the lens subsequent to being transmitted through the spectral filter.

In additional embodiments, the lens is coupled directly or indirectly to the spectral filter.

Provided also is a wand for an intra-oral imaging system, wherein the wand comprises an optical source that generates light, and an imaging device. The imaging device comprises a lens and a sensor coupled to the lens, wherein the wand is coupled wirelessly to the intra-oral imaging system.

In further embodiments, the wand has a proximal end and a distal end, and wherein the imaging device is coupled to the distal end of the wand. The sensor converts light that is incident on the sensor into electrical signals that are digitized and transmitted wirelessly for processing via a processor.

In yet further embodiments, partial data is transmitted wirelessly for processing via the processor.

The invention will now be further described by way of example with reference to the accompanying drawings, in which:
FIG. 1 illustrates a block diagram of a computing and imaging environment that includes an intra-oral imaging system having a wand with an imaging device that has a spectral filter, in accordance with certain embodiments:
FIG. 2 illustrates a block diagram that shows how fluorescence is caused when a wand with the imaging device is used to image the oral cavity of a patient, in accordance with certain embodiments;
FIG. 3 illustrates a block diagram that shows how a spectral filter operates while imaging the oral cavity of a patient, in accordance with certain embodiments;
FIG. 4 illustrates a first flowchart showing operations performed via at least an imaging device in a wand, in accordance with certain embodiments;
FIG. 5 illustrates a second flowchart showing operations performed via at least an imaging device in a wand, in accordance with certain embodiments;
FIG. 6 illustrates an exemplary intra-oral imaging system in which a wand having an imaging device is included, in accordance with certain embodiments; and
FIG. 7 illustrates an exemplary wand in which an imaging device having a spectral filter is included, in accordance with certain embodiments;
FIG. 8 illustrates an exemplary wand that communicates wirelessly with elements included in the housing of an intra-oral imaging system, in accordance with certain embodiments; and
FIG. 9 illustrates a block diagram of a computational system that shows certain elements of an intra-oral imaging system and an imaging device, in accordance with certain embodiments.

Fluorescence of teeth and other structures in the oral cavity of a patient may cause errors in metrology computations on images acquired by an intra-oral imaging device of an intra-oral imaging system. Such metrology computations may attempt to determine measurements of structures within the oral cavity of a patient and may attempt to generate three-dimensional models of teeth from a sequence of images acquired by the intra-oral imaging device. Certain embodiments eliminate the effects of fluorescence caused by teeth and other structures in the oral cavity a patient by providing a spectral filter.

Certain embodiments provide an imaging device having a spectral filter that allows 405 nanometer (nm) wavelengths of light to pass through and blocks all other wavelengths. The 405 nm wavelength is a metrology wavelength used for determining dimensions of structures in the oral cavity of a patient and for determining three-dimensional models of teeth from a sequence of images.

The imaging device is coupled to a wand that has an optical source illuminating the oral cavity of a patient with 405 nm wavelengths of light. The structures such as teeth, tongue, palate, gingiva, etc., that are present in the oral cavity exhibit fluorescence causing spurious wavelengths that may affect metrology used for determining three-dimensional structures of teeth and other structures present in the oral cavity. To avoid errors in metrology, the spectral filter may be used to allow transmittal of only 405 nm wavelengths and images of the oral cavity acquired by the imaging device may be used for metrology. In alternative embodiments, other wavelengths may be used for metrology and other spectral filters may be used.

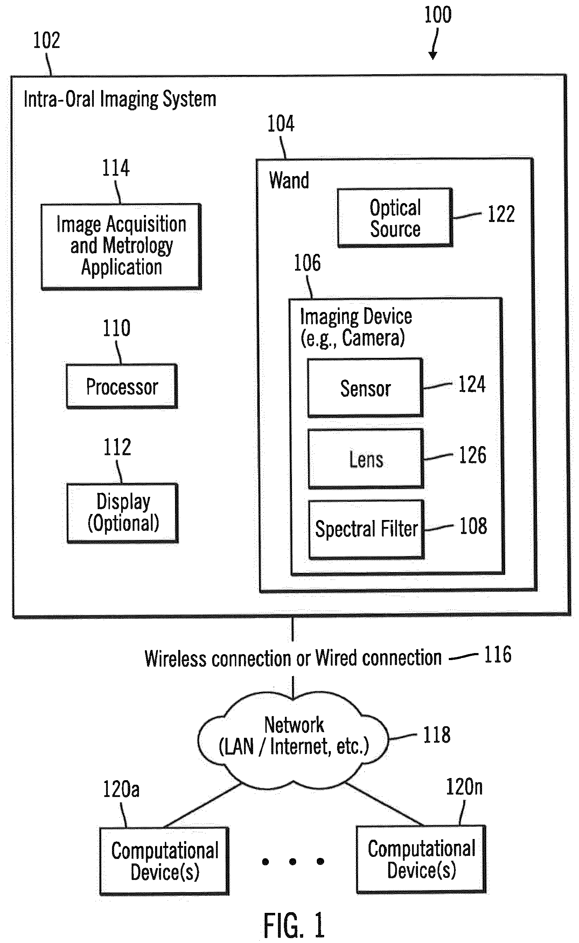

FIG. 1 illustrates a block diagram of a computing and imaging environment 100 that includes an intra-oral imaging system 102 having a wand 104 with an imaging device 106 that has a spectral filter 108, in accordance with certain embodiments.

The intra-oral imaging system 102 is comprised of a processor 110, a display 112, a wand 104, and an image acquisition and metrology application 114. The intra-oral imaging system 102 may be coupled via a wired or wireless connection 116 over a network 118 to one or more computational devices 120a...120n. The computational devices 120a...120n may include any suitable computational device such as a personal computer, a server computer, a mini computer, a mainframe computer, a blade computer, a tablet computer, a touchscreen computing device, a telephony device, a cell phone, a mobile computational device, etc., and some of the computational devices may provide web services or cloud computing services. The network 118 may comprise any suitable network known in the art such as a local area network, an intranet, the Internet, a storage area network, etc.

The wand 104 has an optical source 122 and an imaging device 106, such as, a camera. The imaging device 106 may include a sensor 124, a lens 126 and a spectral filter 108. The optical source 122 may be used for illuminating the oral cavity of a patient and is designed to meet laser safety limits for visible light. In certain embodiments, under control of the image acquisition and metrology application 114 the optical source 122 may generate light of 405 nm wavelength. In certain embodiments, the optical source 122 may generate light at a wavelength that is less than 405 nm. The optical source 122 may be a type of light emitting diode (LED) or some other suitable light source. The 405 nm wavelength is an exemplary wavelength and other exemplary wavelengths may be used in other embodiments. In certain embodiments there is a spectral width to the wavelengths used. For example, in certain embodiments a laser may emit over a 1 nm wide spectrum around 405 nm.

In certain embodiments, the sensor 124 may be comprised of an array of millions of tiny pixels in order to produce a image. The photons of light that fall on the pixels are converted into electrical signals by the sensor 124. The lens 126 is also referred to as camera lens or a photographic lens, and may be an optical lens or assembly of lenses used in conjunction with a camera body and mechanism to acquire digital images of objects. In certain embodiments the lens is a convex lens. The spectral filter 108 that is positioned in front of the lens 126 may be circular, square or oblong or of any other shape. In certain embodiments the spectral filter 108 is a glass or plastic disc with a ring frame that may be screwed in front of or clipped to the lens 126 or may otherwise be placed in front of the lens 126. In other embodiments the spectral filter 108 may be integrated into the lens 126. The light entering the spectral filter 108 may have a spectral distribution that may depend on both the spectral characteristics of the optical source 122 and the fluorescence of the structures of the oral cavity.

A dental practitioner may hold the wand 104 inside a patient's oral cavity, The optical source 122 of the wand 104 illuminates the oral cavity and the imaging device 106 captures a plurality of digital images of structures in the oral cavity, such as the patient's teeth, gingivae, and/or palate. Three-dimensional digital models of the teeth may be generated from the plurality of digital images via metrology performed by the image acquisition and metrology application 114, and the digital models may be viewed on the display 112.

Therefore, FIG. 1 illustrates certain embodiments in which a spectral filter 108 is included in front of the lens 126 of an imaging device 106 of a wand 104 of an intra-oral imaging system 102.

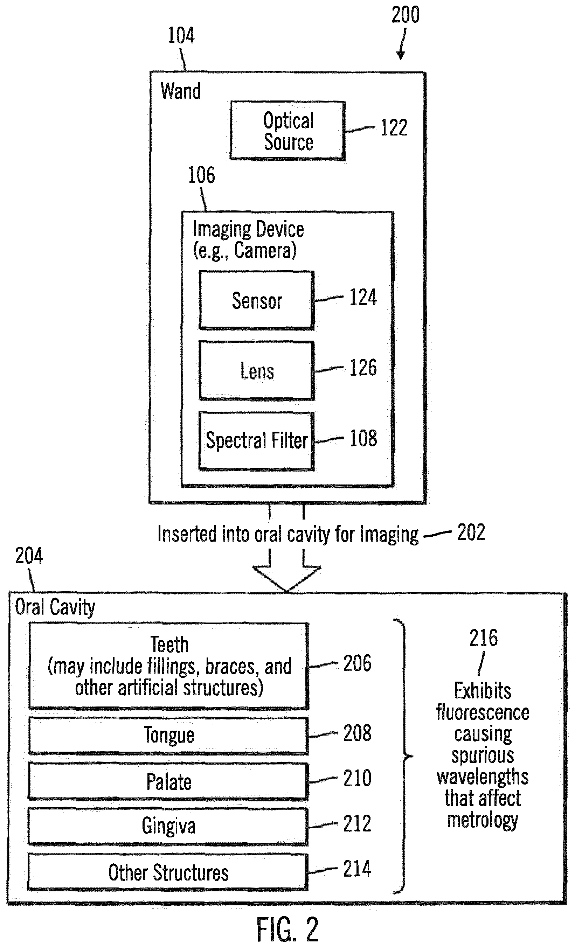

FIG. 2 illustrates a block diagram 200 that shows how fluorescence is caused when the wand 104 with the imaging device 106 is used to image the oral cavity 204 of a patient, in accordance with certain embodiments.

In certain embodiments a dental practitioner inserts (reference numeral 202) the wand 104 into the oral cavity 204 of a patient. The oral cavity 204 of the patient may include structures such as teeth 206, tongue 208, palate 210, gingiva 212, and other structures 214, such as fillings, braces, etc. The structures 206, 208, 210, 212, 214 in the oral cavity 204 may exhibit fluorescence (reference numeral 216) when the oral cavity 204 is illuminated via the light from the optical source 122. Fluorescence is the emission of light by a substance that has absorbed light or other electromagnetic radiation and is a type of luminescence. In many cases, the emitted light caused by fluorescence has a longer or different wavelength, and may have a shallower energy, than the absorbed radiation. For example teeth can exhibit an intense blue fluorescence when ultraviolet light falls on the teeth. When teeth are illuminated with high intensity blue light they may start to emit light in the green part of the spectrum. The fluorescence of the dental material has a direct relation with the mineral content of the enamel. Similarly when teeth are illuminated with violet light (405 nm in wavelength which is the metrology wavelength in certain embodiments) the teeth may exhibit fluorescence. The fluorescence of teeth and other structures within the oral cavity may also be referred to as auto-fluorescence.

However, the fluorescence of structures within the oral cavity introduces errors in metrology operations used for measuring dimensions of structures in the oral cavity and for three-dimensional reconstruction of digital models of teeth.

Therefore, FIG. 2 illustrates certain embodiments that show how fluorescence caused by teeth 206 and other structures in the oral cavity 204 a patient may cause errors in metrology unless the effects of fluorescence are eliminated.

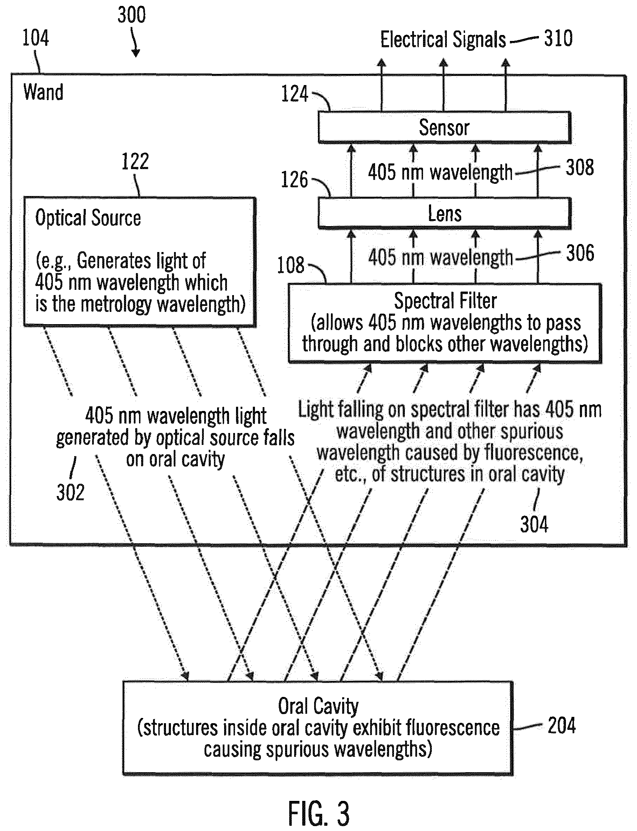

FIG. 3 illustrates a block diagram 300 that show how a spectral filter 108 operates while imaging the oral cavity 204 of a patient, in accordance with certain embodiments.

In certain embodiments, after placing the wand 104 in the oral cavity 204 of a patient, the dental practitioner may press a button on the wand 104 and the image acquisition and metrology application 114 may trigger the optical source 122 to generate light at 405 nm wavelength to illuminate the oral cavity 204 (reference numeral 302) . The metrology operations performed by the image acquisition and metrology application 114 may have been designed for analyzing images of objects illuminated with 405 nm light.

However, structures inside the oral cavity 204 exhibit fluorescence and when the imaging device 106 is used to initiate the capturing of images for metrology, then the light that falls on the spectral filter 108 includes both 405 nm light and light of other wavelengths caused by fluorescence (reference numeral 304).

The spectral filter 108 is designed to allow only 405 nm light to pass through and block all other wavelengths. As a result the light that falls on the lens 126 after passing through the spectral filter 108 includes only 405 nm wavelengths and do not include the spurious wavelengths caused by fluorescence of structures of the oral cavity (reference numeral 306).

The light of 405 nm wavelength passes through the lens 126 and falls on the sensor 124 (reference numeral 308), where photons of the light energize the pixels in the pixel array of the sensor 124 and is converted to electrical signals 310 that are digitally stored by the image acquisition and metrology application 114 in storage media maintained in the intra-oral imaging system 102. In certain embodiments, the image acquisition and metrology application 114 may forward the images to one or more of the computational devices 120a...120n for further processing. The image acquisition and metrology application 114 may process one or more of the images to determine various dimensions of structures of the oral cavity, and to determine three-dimensional models of the structures of the oral cavity and display the models on the display 112 of the intra-oral imaging system 102.

Therefore, FIG. 3 illustrates certain embodiments in which the spectral filter 108 eliminates the effects of the fluorescence of teeth and other structures in metrology computations. While FIG. 3 illustrates that light is first transmitted through the spectral filter 108 and is then transmitted through the lens 126, in alternative embodiments the spectral filter 108 may be placed in between the sensor 124 and the lens 126. In such alternative embodiments, light may first be transmitted through the lens 126 and may then be transmitted through the spectral filter 108, and 405 nm wavelengths may then be incident on the sensor 124.

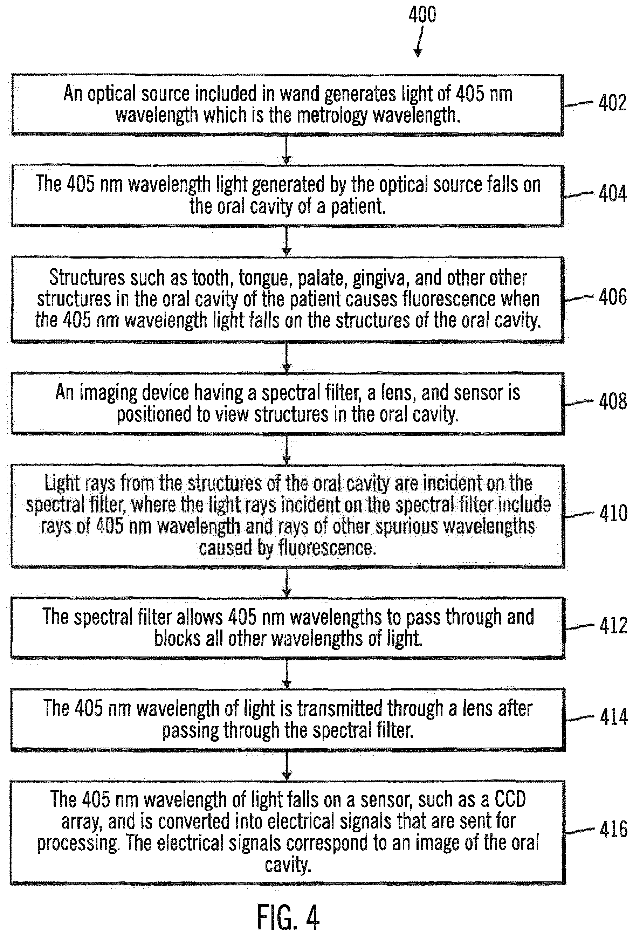

FIG. 4 illustrates a first flowchart 400 that shows operations performed via at least an imaging device 106 in a wand 104, in accordance with certain embodiments. Certain of the operations may be performed under the control of the image acquisition and metrology application 114 executing on the processor 110 of the intra-oral imaging system 102.

Control starts at block 402 in which an optical source 122 included in a wand 104 generates light of 405 nm wavelength which is the metrology wavelength. In other embodiments, light of a different new wavelength may be used for metrology in which case the spectral filter 108 may be designed to transmit light of the new wavelength and block all other wavelengths.

The 405 nm wavelength light generated by the optical source falls (at block 404) on the oral cavity 204 of a patient, Structures such as tooth, tongue, palate, gingiva, and other structures in the oral cavity 204 of the patient causes (at block 406) fluorescence when the 405 nm wavelength light falls on the structures of the oral cavity 204.

An imaging device 106 having a spectral filter 108, a lens 126, and a sensor 124 is positioned (at block 408) to view structures in the oral cavity 204. Control proceeds to block 410 in which light rays from the structures of the oral cavity 204 are incident on the spectral filter 108, where the light rays incident on the spectral filter 108 include rays of 405 nm wavelength and rays of other spurious wavelengths caused by fluorescence.

Control proceeds to block 412 in which the spectral filter 108 allows 405 nm wavelengths to pass through the spectral filter 108 and block all other wavelengths of light. The 405 nm wavelength of light is transmitted (at block 414) through the lens 126 after passing through the spectral filter 108. The 405 nm wavelength of light then falls on the sensor 124 comprising a charge coupled device (CCD) array, and is converted (at block 416) into electrical signals that are sent for processing. The electrical signals correspond to an image of the oral cavity.

Therefore, FIG. 4 illustrates certain operations performed by an intra-oral imaging system 102 to eliminate the effects of fluorescence by using a spectral filter 108.

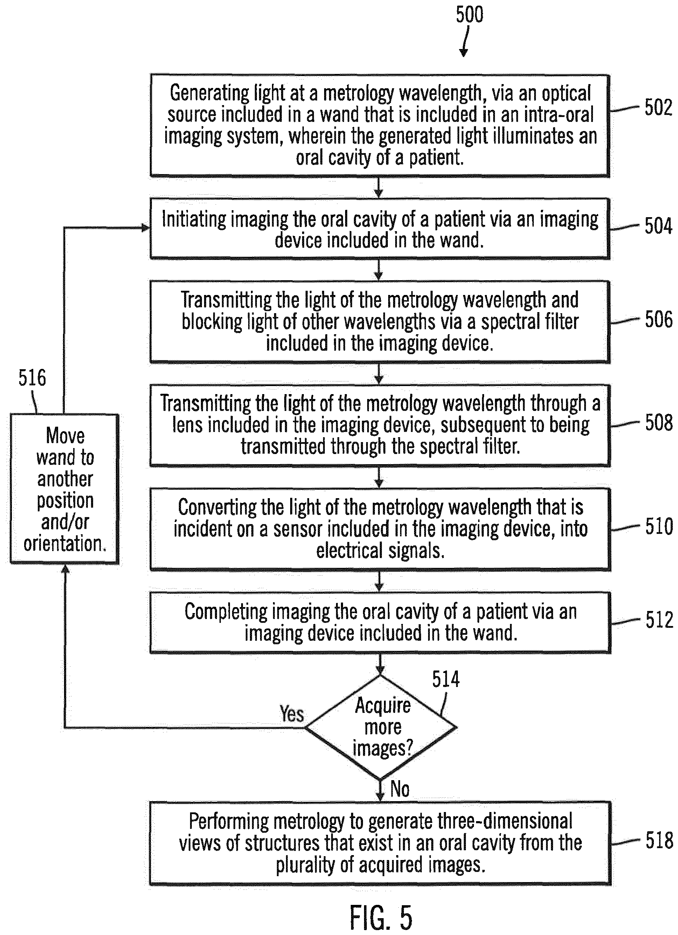

FIG. 5 illustrates a second flowchart 500 that shows operations performed via at least an imaging device 106 in a wand 104 under control of an image acquisition and metrology application 114, in accordance with certain embodiments.

Control starts at block 502 in which the image acquisition and metrology application 114 triggers the optical source 122 to generate light at a metrology wavelength. The optical source 122 is included in a wand 104 that is included in an intra-oral imaging system 102, where the generated light illuminates an oral cavity 204 of a patient.

Control proceeds to block 504 in which the image acquisition and metrology application 114 triggers an initiation of the imaging of the oral cavity 204 of a patient via an imaging device 106 included in the wand 104. In response to the initiation of the imaging of the oral cavity 204, at block 505 the spectral filter 108 included in the imaging device 106 transmits the light of the metrology wavelength and blocks light of other wavelengths.

The light of the metrology wavelength is transmitted (at block 508) through a lens 126 included in the imaging device 106, subsequent to being transmitted through the spectral filter 108. The light on the metrology wavelength that is incident on the sensor 124 included in the imaging device 106 is converted (at block 510) into electrical signals and the process to acquire one or more images of the oral cavity of the patient via the imaging device 106 included in the wand is completed (at block 512).

Control proceeds to block 514 where a determination is made by the image acquisition and metrology application 114 as to whether more images of the oral cavity 204 are to be acquired. If so, control proceeds to block 516 in which the wand may be moved to another position or orientation and control proceeds to block 504 to acquire additional images of the oral cavity.

If at block 514, a determination is made that no more images are to be acquired then control proceeds to block 518 in which the image acquisition and metrology application performs metrology to generate three-dimensional views of structures that are present in an oral cavity 204, from the plurality of acquired images. In certain alternative embodiments the three dimensional structures are shown on the display 112 in real time as more and more sequences of images are captured. In other words, as more and more images keep getting captured, the image acquisition and metrology application 114 may continuously keep on displaying in real-time the corresponding three dimensional models of structures of the oral cavity 204.

Therefore, FIG, 5 illustrates certain embodiments in which the effects of fluorescence are eliminated in metrology computations from intra-oral imagery, by using a spectral filter to filter spurious wavelengths caused by fluorescence.

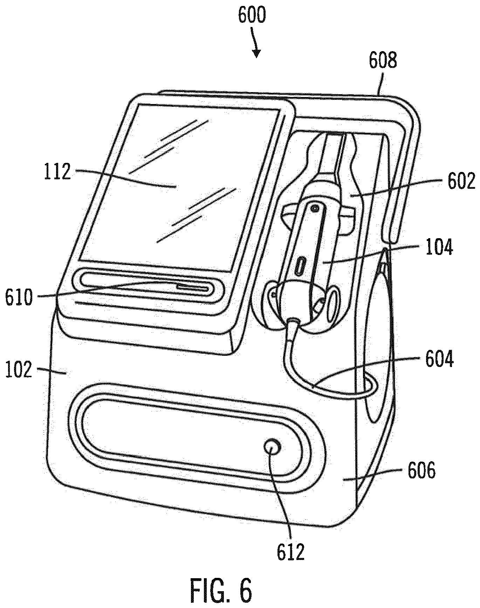

FIG. 6 illustrates a view 600 of the exemplary intra-oral imaging system 102 in which the wand 104 having the imaging device 106 with the spectral filter 108 is included, in accordance with certain embodiments. It should be noted that intra-oral imaging system 102 is exemplary and other intra-oral imaging systems may be used in alternative embodiments.

The intra-oral imaging system 102 may include a wand 104 having an optical source 122 and an imaging device 106. The wand 104 is small and light weight for use by dental practitioners, and the imaging process is fast and simple to use, allowing the imaging of both arches and bites to be accomplished rapidly, such that a digital model of the imaged areas may be viewed on a display 116, where in certain embodiments the display 116 is a touchscreen display.

The intra-oral imaging system 102 may include a wand storage area 602 in which the wand 104 may be stored. The wand 104 may be extensibly coupled via a cord 604 to the housing 606 of the intra-oral imaging system 102.

The intra-oral imaging system 102 may include a handle 608 that may be used for carrying the intra-oral imaging system 102 from one location to another. The handle 608 may also be referred to as a carrying handle.

In addition to the handle 608, the display 112, the wand 104, and the housing 606, the intra-oral imaging system 102 includes a power button 612 that is located on the front face of the intra-oral imaging system 102. The power button 612 may be used to switch the intra-oral imaging system 102 on and off. Additionally, light emitting diode (LED) based indicators 610 may indicate one or more status related to the operational state of the 3D intra-oral imaging system 102.

Therefore, FIG. 6 illustrates certain embodiments in which an intra-oral imaging system 102 includes a wand 104 that includes an imaging device 106 with a spectral filter 108.

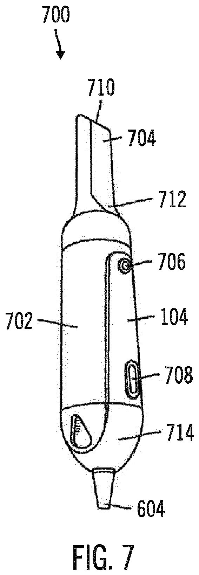

FIG. 7 illustrates a view 700 of a wand 104 in which the imaging device 106 having the spectral filter 108 is included, in accordance with certain embodiments. Components of the wand 104 may be wholly or partially enclosed within an housing 702 that protects the optical components of the wand 104 from dust and debris to maintain measurement accuracy.

The tip 704 is the portion of the wand 104 that is inserted into a patient's mouth. The imaging device 106 and the optical source 122 may be embedded within the tip 704 of the wand. The tip 704 of the wand 104 may include an optical window 710 made of biocompatible, transparent material that may be either plastic or glass. The optical window 710 may be mounted into the plastic tip housing such that no sharp corners or edges contact human tissue. The light from the optical source 122 is transmitted through the optical window 710, and the imaging device 106 captures images of the structures of the oral cavity 204 through the optical window 710. It should be emphasized that the wand tip 704 is designed to be long enough to reach the back teeth of a typical patient.

The wand 104 has a round molded area in which there is a switch 706 that when pressed by a dental practitioner can start recording of images on and off. In the oval molded area 708 there are keypad buttons to traverse through items from the graphical user interface displayed on the display 112. In certain embodiments, the tip 404 of the wand 104 is covered with a disposable molded plastic sheath that snaps on and off the wand 104. The disposable molded plastic sheath may be transparent and may have a mirror.

The end comprising the tip 704 of the wand 104 may be referred to as the distal end 712 of the wand and the end to which the cord 604 is extensibly coupled may be referred to as the proximal end 714 of the wand 104.

Therefore, FIGS. 1-7 illustrate certain embodiments in which the effects of fluorescence of teeth and other structures of an oral cavity 204 are eliminated by applying a spectral filter 108 before light passes to the lens 126 of an imaging device 106 of a wand 104 of an intra-oral imaging system 102.

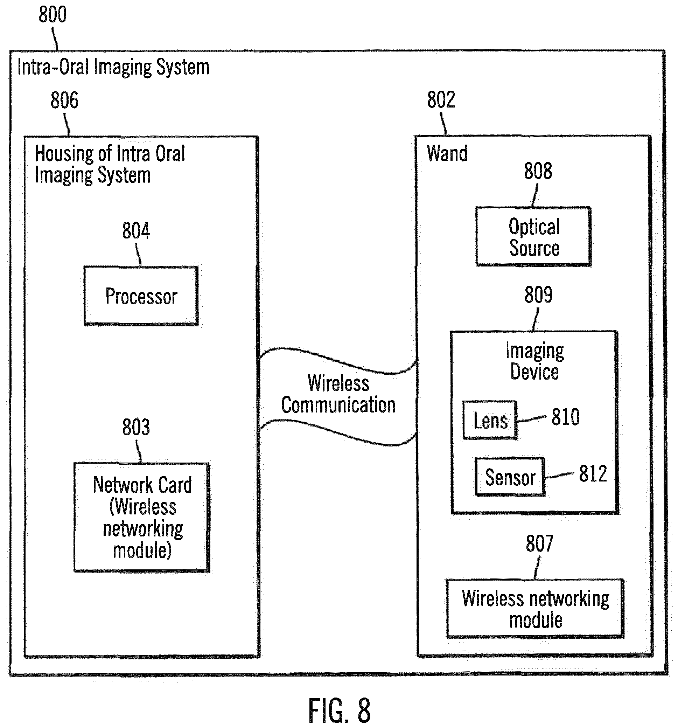

FIG. 8 illustrates a block diagram of an intra-oral imaging system 800 that includes an exemplary wand 802 that communicates wirelessly with elements (e.g. network card 803, processor 804, etc.) included in the housing 806 of the intra-oral imaging system 800, in accordance with certain embodiments. The intra-oral imaging system 800 shown in FIG. 8 may externally appear similar to intra-oral imaging system shown in FIG. 6 except for the absence of the cord 604.

In certain embodiments, the wand 802 comprises a wireless networking module 807, an optical source 808 that generates light, and an imaging device 809. The imaging device 809 comprises a lens 810 and a sensor 812 coupled to the lens 810, wherein the wand 802 is coupled wirelessly to elements of the housing 806 of the intra-oral imaging system 800.

In further embodiments, the wand 802 has a proximal end and a distal end, wherein the imaging device 809 is coupled to the distal end of the wand 802. The proximal end of the wand 802 may he held by a dental practitioner. The sensor 812 converts light that is incident on the sensor 812 into electrical signals that are digitized and transmitted wirelessly to the network card 803 for processing via the processor 804. In certain embodiments, the network card 803 is a wireless networking module configured for wireless communication with the wand 802 via the wireless networking module 807 included in the wand 802. In certain embodiments, only partial data may be transmitted wirelessly from the wand 802 as the overall data collected by the imaging device 809 may be too time consuming to transmit wirelessly.

In certain embodiments, the intra-oral imaging system 800 may perform the operations with spectral filters and spectral filtering that are shown in FIGS. 1-7.

Therefore, FIGS. 1-8 illustrate certain embodiments in which the effects of fluorescence of teeth and other structures of an oral cavity 204 are eliminated by applying a spectral filter 108 before light passes to the lens 126 of an imaging device 106 of a wand 104 of an intra-oral imaging system 102.

The operations described in FIGS. 1-8 may be implemented as a method, apparatus or computer program product using techniques to produce software, firmware, hardware, or any combination thereof. Additionally, certain embodiments may take the form of a computer program product embodied in one or more computer readable storage medium(s) having computer readable program code embodied therein.

A computer readable storage medium may include an electronic, magnetic, optical, electromagnetic, or semiconductor system, apparatus, or device, or any suitable combination of the foregoing. The computer readable storage medium may also comprise an electrical connection having one or more wires, a portable computer diskette or disk, a hard disk, a random access memory (RAM), a read-only memory (ROM), an erasable programmable read-only memory (EPROM or Flash memory), a portable compact disc read-only memory (CD-ROM), an optical storage device, a magnetic storage device, etc. A computer readable storage medium may be any tangible medium that can contain, or store a program for use by or in connection with an instruction execution system, apparatus, or device.

Computer program code for carrying out operations for aspects of the present invention may be written in any combination of one or more programming languages.

Features of the present invention are described below with reference to flowchart illustrations and/or block diagrams of methods, system and computer program products according to certain embodiments. At least certain operations that may have been illustrated in the figures show certain events occurring in a certain order. In alternative embodiments, certain operations may be performed in a different order, modified or removed. Additionally, operations may be added to the above described logic and still conform to the described embodiments. Further, operations described herein may occur sequentially or certain operations may be processed in parallel. Yet further, operations may be performed by a single processing unit or by distributed processing units. Computer program instructions can implement the blocks of the flowchart. These computer program instructions may be provided to a processor of a computer for execution.

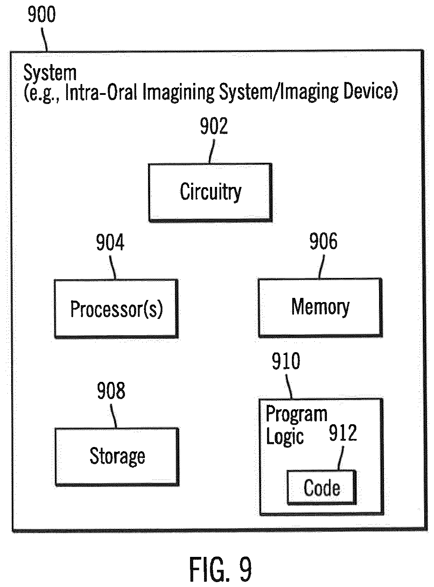

FIG. 9 illustrates a block diagram that shows certain elements that may be included in the intra-oral imaging system 102 or the imaging device 106 or any of the computational devices 120a...120n, in accordance with certain embodiments. The system 900 may comprise intra-oral imaging system 102 or the imaging device 106 or the computational devices 120a... 120n and may include a circuitry 902 that may in certain embodiments include at least a processor 904, such as the processor 110. The system 900 may also include a memory 906 (e.g., a volatile memory device), and storage 908. The storage 908 may include a non-volatile memory device (e.g., EEPROM, ROM, PROM, RAM, DRAM, SRAM, flash, firmware, programmable logic, etc.), magnetic disk drive, optical disk drive, tape drive, etc. The storage 908 may comprise an internal storage device, an attached storage device and/or a network accessible storage device. The system 900 may include a program logic 910 including code 912 that may be loaded into the memory 906 and executed by the processor 904 or circuitry 902. In certain embodiments, the program logic 910 including code 912 may be stored in the storage 908. In certain other embodiments, the program logic 910 may be implemented in the circuitry 902. Therefore, while FIG. 9 shows the program logic 910 separately from the other elements, the program logic 910 may be implemented in the memory 906 and/or the circuitry 902.

The terms "an embodiment", "embodiment", "embodiments", "the embodiment", "the embodiments", "one or more embodiments", "some embodiments", and "one embodiment" mean "one or more (but not all) embodiments of the present invention(s)" unless expressly specified otherwise.

The terms "including", "comprising", "having" and variations thereof mean "including but not limited to", unless expressly specified otherwise.

The enumerated listing of items does not imply that any or all of the items are mutually exclusive, unless expressly specified otherwise.

The terms "a", "an" and "the" mean "one or more", unless expressly specified otherwise.

Devices that are in communication with each other need not be in continuous communication with each other, unless expressly specified otherwise. In addition, devices that are in communication with each other may communicate directly or indirectly through one or more intermediaries.

A description of an embodiment with several components in communication with each other does not imply that all such components are required. On the contrary a variety of optional components are described to illustrate the wide variety of possible embodiments.

When a single device or article is described herein, it will be readily apparent that more than one device/article (whether or not they cooperate) may be used in place of a single device/article. Similarly, where more than one device or article is described herein (whether or not they cooperate), it will be readily apparent that a single device/article may be used in place of the more than one device or article or a different number of devices/articles may be used instead of the shown number of devices or programs. The functionality and/or the features of a device may be alternatively embodied by one or more other devices which are not explicitly described as having such functionality/features.

The foregoing description of various embodiments of the invention has been presented for the purposes of illustration and description. It is not intended to be exhaustive or to limit the invention to the precise form disclosed. Many modifications and variations are possible in light of the above teaching.

## Claims

1. An imaging device (106, 809) coupled to a wand having an optical source (122, 808) that generates light at a metrology wavelength, wherein the wand is included in an intra-oral imaging system (102, 800), the imaging device (106, 809) comprising a spectral filter (108) that transmits the light of the metrology wavelength and blocks light of other wavelengths a lens (126, 810) coupled to the spectral filter (108), and a sensor (124, 812), wherein the light of the metrology wavelength is incident on the sensor after being transmitted through the lens (126, 810) and the spectral filter (108), and wherein the sensor converts the light of the metrology wavelength that is incident on the sensor into electrical signals to generate intra-oral imagery, **characterised in that** the metrology wavelength is 405 +/-1 nanometers or less; and **in that** the intra-oral imaging system (102, 800) is configured to perform metrology by capturing a plurality of images with the imaging device (106, 809) to generate three-dimensional views of structures that exist in an oral cavity.

2. The imaging device (106, 809) of claim 1, wherein the light of other wavelengths are caused via fluorescence of the structures that exist in the oral cavity, wherein the fluorescence includes auto-fluorescence of teeth, and wherein removal of the spectral filter (108) from the imaging device (106, 809) causes errors in generation of the three-dimensional structures because the other wavelengths are spurious wavelengths that are unsuitable for performing the metrology.

3. The imaging device (106, 809) in either claim 1 or claim 2, wherein the wand has a proximal end and a distal end, and wherein the imaging device (106, 809) is coupled to the distal end of the wand, and the intra-oral imaging system (102, 800) is configured to digitize and wirelessly transmit the electrical signals for processing via a processor.

4. The imaging device (106, 809) of any preceding claim, wherein the imaging device (106, 809) is a digital camera, and wherein the spectral filter (108) is removable from the imaging device (106, 809).

5. The imaging device (106, 809) of any preceding claim, configured to transmit the light of the metrology wavelength through the lens (126, 810) subsequent to being transmitted through the spectral filter (108).

6. The imaging device (106, 809) of any preceding claim, wherein the lens (126, 810) is coupled directly or indirectly to the spectral filter (108).

7. A wand for an intra-oral imaging system (102, 800), the wand comprising, an optical source (122, 808) that generates light at a metrology wavelength of 405 +/-1 nanometers, and the imaging device (106, 809) of any preceding claim.

8. An intra-oral imaging system (102, 800), comprising a processor (804, 904), a housing (606, 806) encasing the processor and the wand (802) of claim 7, extensibly or wirelessly coupled to the housing (606, 806) for capturing images.

9. A computer readable storage medium wherein code embodied in the computer readable storage medium when executed by a processor performs operations in the intra-oral imaging system (102, 800) of claim 1, the operations comprising:
generating light at a metrology wavelength, via an optical source (122, 808) included in a wand that is included in the intra-oral imaging system (102, 800), wherein the generated light illuminates an oral cavity of a patient, and
imaging the oral cavity of a patient via an imaging device (106, 809) included in the wand, wherein the imaging of the oral cavity of the patient, comprises:
transmitting the light of the metrology wavelength and blocking light of other wavelengths via a spectral filter (108) included in the imaging device (106, 809);
transmitting at least the light of the metrology wavelength through a lens (126, 810) included in the imaging device (106, 809); and
converting the light of the metrology wavelength that is incident on a sensor (124, 812) included in the image device after being transmitted through the lens (126, 810) and the spectral filter (108) into electrical signals to generate intra-oral imagery,
**characterised in that** the metrology wavelength is 405 +/-1 nanometers, and **in that** the operations further comprise:
performing metrology by capturing a plurality of images by the imaging device (106, 809) to generate three-dimensional views of structures that exist in an oral cavity of a patient.

10. A wand (104, 802) for an intra-oral imaging system (102, 800), the wand (104, 802) comprising an optical source (122, 808) that generates light, and the imaging device (106, 809), of any one of claims 1 to 6, wherein the wand (104, 802) is coupled wirelessly to the intra-oral imaging system (102, 800).

11. The wand (104, 802) of claim 10, wherein the wand (104, 802) has a proximal end and a distal end, and wherein the imaging device (106, 809) is coupled to the distal end of the wand (104, 802), and the sensor converts light that is incident on the sensor into electrical signals that are digitized and transmitted wirelessly for processing via a processor (110, 804, 904).

12. The wand (104, 802) of claim 11, wherein partial data is transmitted wirelessly for processing via the processor (110, 804, 904).

## Patentansprüche

1. Bildgebungsvorrichtung (106, 809), die mit einem Handstück gekoppelt ist, das eine Lichtquelle hat, die Licht mit einer metrologischen Wellenlänge erzeugt, wobei das Handstück in einem intraoralen Bildgebungssystem (102, 800) enthalten ist, wobei die Bildgebungsvorrichtung (106, 809) ein Spektralfilter (108), das das Licht der metrologischen Wellenlänge durchlässt und das Licht anderer Wellenlängen blockiert, eine Linse (126, 810), die mit dem Spektralfilter (108) gekoppelt ist, und einen Sensor (124, 812) aufweist, wobei das Licht der metrologischen Wellenlänge auf den Sensor fällt, nachdem es durch die Linse (126, 810) und das Spektralfilter (108) durchgelassen worden ist, und wobei der Sensor das auf den Sensor fallende Licht der metrologischen Wellenlänge in elektrische Signale zum Erzeugen intraoraler Bilder umwandelt, **dadurch gekennzeichnet, dass** die metrologische Wellenlänge 405 ± 1 Nanometer oder kleiner ist; und dadurch, dass das intraorale Bildgebungssystem (102, 800) zum Durchführen von Metrologie durch Erfassen einer Mehrzahl von Bildern mit der Bildgebungsvorrichtung (106, 809) zum Erzeugen dreidimensionaler Ansichten von Strukturen, die in einer Mundhöhle vorhanden sind, konfiguriert ist.

2. Bildgebungsvorrichtung (106, 809) nach Anspruch 1, wobei das Licht anderer Wellenlänge über Fluoreszenz der Strukturen, die in der Mundhöhle vorhanden sind, verursacht wird, wobei die Fluoreszenz Autofluoreszenz von Zähnen enthält und wobei das Entfernen des Spektralfilters (108) von der Bildgebungsvorrichtung (106, 809) Fehler bei der Erzeugung der dreidimensionalen Strukturen verursacht, weil die anderen Wellenlängen störende Wellenlängen sind, die für die Durchführung der Metrologie ungeeignet sind.

3. Bildgebungsvorrichtung (106, 809) nach Anspruch 1 oder Anspruch 2, wobei das Handstück ein proximales Ende und ein distales Ende hat und wobei die Bildgebungsvorrichtung (106, 809) mit dem distalen Ende des Handstücks gekoppelt ist und das intraorale Bildgebungssystem (102, 800) zum Digitalisieren und drahtlosen Übertragen der elektrischen Signale zur Verarbeitung durch einen Prozessor konfiguriert ist.

4. Bildgebungsvorrichtung (106, 809) nach einem der vorhergehenden Ansprüche, wobei die Bildgebungsvorrichtung (106, 809) eine digitale Kamera ist und wobei das Spektralfilter (108) aus der Bildgebungsvorrichtung (106, 809) entfernt werden kann.

5. Bildgebungsvorrichtung (106, 809) nach einem der vorhergehenden Ansprüche, die zum Durchlassen des Lichts der metrologischen Wellenlänge durch die Linse (126, 810), nachdem es durch das Spektralfilter (108) durchgelassen worden ist, konfiguriert ist.

6. Bildgebungsvorrichtung (106, 809) nach einem der vorhergehenden Ansprüche, wobei die Linse (126, 810) direkt oder indirekt mit dem Spektralfilter (108) gekoppelt ist.

7. Handstück für ein intraorales Bildgebungssystem (102, 800), wobei das Handstück eine Lichtquelle (122, 808), die Licht mit einer metrologischen Wellenlänge von 405 ± 1 Nanometer erzeugt, und die Bildgebungsvorrichtung (106, 809) nach einem der vorhergehenden Ansprüche aufweist.

8. Intraorales Bildgebungssystem (102, 800), das einen Prozessor (804, 904), ein den Prozessor umschließendes Gehäuse (606, 806) und das ausziehbar oder drahtlos mit dem Gehäuse (606, 806) gekoppelte Handstück (802) nach Anspruch 7 zum Erfassen von Bildern aufweist.

9. Computerlesbares Speichermedium, wobei im computerlesbaren Speichermedium eingebetteter Code bei Ausführung durch einen Prozessor Vorgänge im intraoralen Bildgebungssystem (102, 800) nach Anspruch 1 durchführt, wobei die Vorgänge Folgendes aufweisen:
Erzeugen von Licht mit einer metrologischen Wellenlänge durch eine Lichtquelle (122, 808), die in einem Handstück enthalten ist, das im intraoralen Bildgebungssystem (102, 800) enthalten ist, wobei das erzeugte Licht eine Mundhöhle eines Patienten beleuchtet, und
Abbilden der Mundhöhle eines Patienten durch eine im Handstück enthaltene Bildgebungsvorrichtung (106, 809), wobei das Abbilden der Mundhöhle des Patienten Folgendes aufweist:
Durchlassen des Lichts der metrologischen Wellenlänge und Blockieren von Licht anderer Wellenlängen durch ein in der Bildgebungsvorrichtung (106, 809) enthaltenes Spektralfilter (108);
Durchlassen von wenigstens dem Licht der metrologischen Wellenlänge durch eine in der Bildgebungsvorrichtung (106, 809) enthaltene Linse (126, 810) und
Umwandeln des Lichts der metrologischen Wellenlänge, das auf einen in der Bildgebungsvorrichtung enthaltenen Sensor (124, 812) fällt, nachdem es durch die Linse (126, 810) und das Spektralfilter (108) durchgelassen worden ist, in elektrische Signale zum Erzeugen von intraoralen Bildern,
**dadurch gekennzeichnet, dass** die metrologische Wellenlänge 405 ± 1 Nanometer ist, und dadurch, dass die Vorgänge ferner Folgendes aufweisen:
Durchführen von Metrologie durch Erfassen einer Mehrzahl von Bildern mit der Bildgebungsvorrichtung (106, 809) zum Erzeugen dreidimensionaler Ansichten von Strukturen, die in einer Mundhöhle vorhanden sind.

10. Handstück (104, 802) für ein intraorales Bildgebungssystem (102, 800), wobei das Handstück (104, 802) eine Licht erzeugende Lichtquelle (122, 808) und die Bildgebungsvorrichtung (106, 800) nach einem der Ansprüche 1 bis 6 aufweist, wobei das Handstück (104, 802) drahtlos mit dem intraoralen Bildgebungssystem (102, 800) gekoppelt ist.

11. Handstück (104, 802) nach Anspruch 10, wobei das Handstück (104, 802) ein proximales Ende und ein distales Ende hat und wobei die Bildgebungsvorrichtung (106, 809) mit dem distalen Ende des Handstücks (104, 802) gekoppelt ist und der Sensor Licht, das auf den Sensor fällt, in elektrische Signale umwandelt, die zur Verarbeitung durch einen Prozessor (110, 804, 904) digitalisiert und drahtlos übertragen werden.

12. Handstück (104, 802) nach Anspruch 11, wobei teilweise Daten zur Verarbeitung durch den Prozessor (110, 804, 904) drahtlos übertragen werden.

## Revendications

1. Appareil d'imagerie (106, 809) couplé à une baguette ayant une source optique (122, 808) qui génère de la lumière à une longueur d'onde de métrologie, où la baguette est incluse dans un système d'imagerie intraorale (102, 800), l'appareil d'imagerie (106, 809) comprenant un filtre spectral (108) qui transmet la lumière de la longueur d'onde de métrologie et bloque la lumière d'autres longueurs d'onde, une lentille (126, 810) couplée au filtre spectral (108), et un capteur (124, 812), où la lumière de la longueur d'onde de métrologie est incidente sur le capteur après avoir été transmise à travers la lentille (126, 810) et le filtre spectral (108), et où le capteur convertit la lumière de la longueur d'onde de métrologie qui est incidente sur le capteur en signaux électriques pour générer une imagerie intraorale, **caractérisé en ce que** la longueur d'onde de métrologie est de 405 +/-1 nanomètres ou moins ; et **en ce que** le système d'imagerie intraorale (102, 800) est configuré pour effectuer la métrologie en saisissant une pluralité d'images avec l'appareil d'imagerie (106, 809) pour générer des vues tridimensionnelles de structures qui existent dans une cavité orale.

2. Appareil d'imagerie (106, 809) selon la revendication 1, dans lequel la lumière d'autres longueurs d'onde est causée par la fluorescence des structures qui existent dans la cavité orale, où la fluorescence comprend l'auto-fluorescence des dents, et où l'enlèvement du filtre spectral (108) de l'appareil d'imagerie (106, 809) cause des erreurs dans la génération des structures tridimensionnelles parce que les autres longueurs d'onde sont des longueurs d'onde parasites qui ne conviennent pas pour effectuer la métrologie.

3. Appareil d'imagerie (106, 809) selon la revendication 1 ou la revendication 2, dans lequel la baguette a une extrémité proximale et une extrémité distale, et où l'appareil d'imagerie (106, 809) est couplé à l'extrémité distale de la baguette, et le système d'imagerie intraorale (102, 800) est configuré pour convertir en numérique et transmettre sans fil les signaux électriques pour traitement par un processeur.

4. Appareil d'imagerie (106, 809) selon l'une quelconque des revendications précédentes, où l'appareil d'imagerie (106, 809) est une caméra numérique et où le filtre spectral (108) est enlevable de l'appareil d'imagerie (106, 809).

5. Appareil d'imagerie (106, 809) selon l'une quelconque des revendications précédentes, configuré pour transmettre la lumière de la longueur d'onde de métrologie à travers la lentille (126, 810) après qu'elle eut été transmise à travers le filtre spectral (108).

6. Appareil d'imagerie (106, 809) selon l'une quelconque des revendications précédentes, dans lequel la lentille (126, 810) est couplée directement ou indirectement au filtre spectral (108).

7. Baguette pour système d'imagerie intraorale (102, 800), la baguette comprenant : une source optique (122, 808) qui génère de la lumière à une longueur d'onde de métrologie de 405 +/-1 nanomètres, et l'appareil d'imagerie (106, 809) selon l'une quelconque des revendications précédentes.

8. Système d'imagerie intraorale (102, 800), comprenant un processeur (804, 904), un boîtier (606, 806) renfermant le processeur et la baguette (802) selon la revendication 7 couplée extensiblement ou sans fil au boîtier (606, 806) pour saisir des images.

9. Support de stockage lisible par ordinateur où du code incorporé dans le support de stockage lisible par ordinateur lorsque exécuté par un processeur, effectue des opérations dans le système d'imagerie intraorale (102, 800) selon la revendication 1, les opérations comprenant :
générer de la lumière à une longueur d'onde de métrologie, par une source optique (122, 808) incluse dans une baguette qui est incluse dans le système d'imagerie intraorale (102, 800), où la lumière générée éclaire une cavité orale d'un patient, et
représenter en imagerie la cavité orale d'un patient au moyen d'un appareil d'imagerie (106, 809) inclus dans la baguette, où l'imagerie de la cavité orale du patient comprend :
transmettre la lumière de la longueur d'onde de métrologie et bloquer la lumière d'autres longueurs d'onde au moyen d'un filtre spectral (108) inclus dans l'appareil d'imagerie (106, 809) ;
transmettre au moins la lumière de la longueur d'onde de métrologie à travers une lentille (126, 800) incluse dans l'appareil d'imagerie (106, 809) ; et
convertir la lumière de la longueur d'onde de métrologie qui est incidente sur un capteur (124, 812) inclus dans l'appareil d'imagerie après qu'elle eut été transmise à travers la lentille (126, 810) et le filtre spectral (108) en signaux électriques pour générer l'imagerie intraorale,
**caractérisé en ce que** la longueur d'onde de métrologie est de 405 +/-1 nanomètres, et **en ce que** les opérations comprennent en outre :
effectuer la métrologie en saisissant une pluralité d'images par l'appareil d'imagerie (106, 809) pour générer des vues tridimensionnelles de structures qui existent dans une cavité orale d'un patient.

10. Baguette (104, 802) pour un système d'imagerie intraorale (102, 800), la baguette (104, 802) comprenant une source optique (122, 808) qui génère de la lumière, et l'appareil d'imagerie (106, 809), selon l'une quelconque des revendications 1 à 6, où la baguette (104, 802) est couplée sans fil au système d'imagerie intraorale (102, 800).

11. Baguette (104, 802) selon la revendication 10, où la baguette (104, 802) a une extrémité proximale et une extrémité distale, et où l'appareil d'imagerie (106, 809) est couplé à l'extrémité distale de la baguette (104, 802), et le capteur convertit la lumière qui est incidente sur le capteur en signaux électriques qui sont convertis en numérique et transmis sans fil pour traitement par un processeur (110, 804, 904).

12. Baguette (104, 802) selon la revendication 11, où des données partielles sont transmises sans fil pour traitement par le processeur (110, 804, 904).
